# EUROPEAN PATENT APPLICATION

(11) **EP 4 813 200 A1**
(43) Date of publication of application: **30.09.2026**
(21) Application number: 25167018.8
(22) Date of filing: 28.03.2025
(51) Int. Cl.: A01H 5/06, A01H 6/82

(54) **POTATOES WITH HIGH POLYPHENOL ANTIOXIDANT CONTENT**

(71) Applicant: IPR B.V., 8501 XG Joure (NL)
(72) Inventor: Oome, Stan, 8501 XG Joure (NL)
(74) Representative: Arnold & Siedsma

(57) **Abstract**

The present invention relates to a potato tuber, comprising a flesh colour ranging from white to dark yellow and an increased concentration of polyphenol antioxidants, in particular flavonoids and/or chlorogenic acid. The phenotype comprising a combination of a flesh colour ranging from white to dark yellow and an increased concentration of polyphenol antioxidants is the result of a genotype comprising a gene encoding a tuber anthocyanin pathway activating transcription factor, in particular an R2R3-MYB transcription factor, and lacking a functional flavonoid 3',5'-hydroxylase (F3'5'H) gene and lacking functional dihydrokaempferol-using alleles of a dihydroflavonol 4-reductase (DFR) gene. The invention further relates to food products comprising the potato tuber and to methods for producing the potato tubers and the plant producing the tubers and a method for selecting potato plants producing the tuber.

## Description

The present invention relates to potato tubers with health benefits. The invention further relates to potato plants producing these tubers, food products comprising these tubers and methods for producing potato plants with health benefits and for producing the healthy potato tubers.

Potatoes are a good source of essential nutrients, such as vitamin C, folate, potassium, and B vitamins and of dietary fibres, which aid digestion. Potatoes are a carbohydrate-rich food and provide a significant amount of energy, which makes them a staple in many diets around the world. Red and purple-fleshed potatoes are naturally abundant in flavonoids and polyphenols, which are key antioxidants that may support overall health. Potato tubers, especially the ones with red and purple coloured flesh, present a very significant source of antioxidants in human nutrition.

Antioxidants play a crucial role in neutralizing harmful free radicals, which are unstable molecules that can cause oxidative stress and damage cells. This oxidative stress is associated with ageing and various diseases, including cancer, heart disease, high blood pressure, and diabetes. By neutralizing free radicals, antioxidants help reduce inflammation in the body. Chronic inflammation is linked to numerous health issues, and consuming antioxidant-rich foods may mitigate this risk. Moreover, antioxidants may support the immune system by protecting cells from damage, thereby enhancing the body's defense against infections and illnesses. Incorporating a variety of antioxidant-rich foods into diet may contribute to overall health and well-being.

Polyphenols are a diverse group of beneficial plant compounds with antioxidant properties. A major subclass of the polyphenols is formed by the flavonoids, which are plant secondary metabolites with a polyphenolic structure having antioxidant activity. These compounds have a broad spectrum of health-promoting effects, including their ability to reduce oxidative stress and inflammation, such as protecting the cardiovascular system by improving endothelial function and reducing the risk of atherosclerosis. Flavonoids enhance insulin sensitivity, exerting antidiabetic effects, while also playing a role in weight regulation by modulating fat metabolism and reducing adiposity. Furthermore, they have been shown to inhibit tumor cell proliferation, highlighting their potential anticancer properties. A part of these health benefits can be ascribed to the interaction and modulation of the gut microbiome. Flavonoids can modulate the microbial composition and activity of the gut thereby enhancing nutrient absorption and metabolic regulation which plays a crucial role in overall health.

The antioxidant chlorogenic acid is found in many plant-based foods and has been reported to positively influence metabolic processes. It exerts a positive effect on glucose and lipid metabolism regulation and on the related disorders, e.g. diabetes, cardiovascular disease (CVD), obesity, certain cancers, and hepatic steatosis. By modulating enzyme activity and metabolic pathways, chlorogenic acid helps regulate blood sugar levels, reduce fat accumulation, and mitigate inflammation, all factors that are essential for maintaining metabolic health.

In plants, chlorogenic acid contributes to disease resistance. It acts as a natural protective agent, contributing to disease resistance by serving as a chemical barrier against pathogens. By strengthening the plant's innate immune response, chlorogenic acid helps prevent infections and enhances overall crop resilience, making it an important compound not only for human health but also agricultural practices.

Whereas many flavonoids are colourless, the best known are probably the anthocyanins, as these give colour to many flowers, fruits and other plant organs, including tubers. In cultivated potatoes there are two main anthocyanin-based (flesh) colours: red and purple. The red-fleshed varieties mostly contain pelargonidins, while the purple varieties mainly contain petunidins.

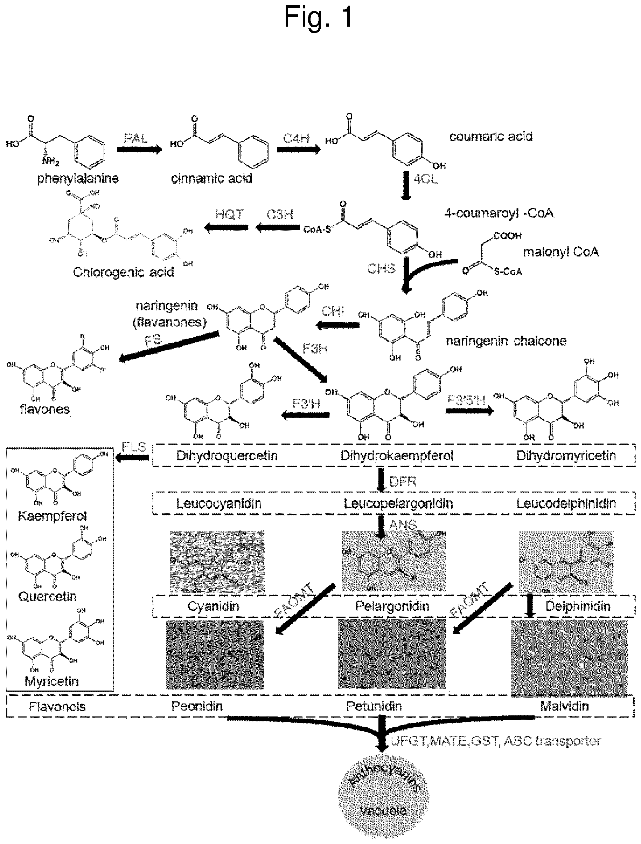

The pathways for both compound groups are identical up to the intermediate dihydrokaempferol (DHK) (**Fig. 1**) ), a flavonoid precursor formed through the action of flavonoid biosynthetic enzymes. At this point, a critical enzymatic modification occurs leading the pathway towards either red or purple anthocyanins.

The enzyme flavonoid-3',5'-hydroxylase (F3'5'H), a cytochrome P450 monooxygenase, catalyzes the hydroxylation of DHK at both the 3' and 5' positions, leading to the formation of dihydromyricetin (DHM). This modification is essential as it introduces two additional hydroxyl groups, which differentiate the pathway leading to petunidin from the one leading to pelargonidin. Following this, the enzyme dihydroflavonol 4-reductase (DFR) catalyzes the reduction of DHM to leucodelphinidin, a key intermediate in the production of purple anthocyanins. Leucodelphinidin is then further processed through a series of additional reactions, ultimately yielding the purple pigments that characterize purple-fleshed potatoes.

On the other hand, in red-fleshed varieties, the absence of the F3'5'H activity prevents the formation of DHM, and the pathway proceeds via an allele of DFR that is capable of using DHK as a substrate, leading to the accumulation of pelargonidin, which imparts the red color. Thus, the specific enzymatic activities of F3'5'H and DFR serve as the regulatory points that direct the biosynthetic pathway towards distinct anthocyanin end-products, determining whether the potato tuber exhibits red or purple flesh. This biochemical distinction in anthocyanin composition underlies the visible color differences observed across different cultivated potato varieties.

In the research leading to the present invention, it was found that it is possible to divert the anthocyanin pathway from the coloured anthocyanins that are responsible for the characteristic red or purple hues in potato flesh, towards the colourless flavonols like kaempferol and its derivatives. This shift in the pathway occurs as a result of changes in the activity of specific enzymes that control hydroxylation, reduction, and other critical modifications in the flavonoid synthesis process. This redirection leads to an accumulation of these flavonols in the potato tuber and a reduction or complete absence of anthocyanin pigments, explaining why the colour of the flesh of these tubers ranges from white to dark yellow instead of red or purple.

White- or yellow-fleshed potato tubers with an altered content of polyphenol antioxidants, like flavonoids and chlorogenic acid, would be a novel class of functional foods with potentially multiple health benefits.

It is therefore the object of the present invention to provide potato tubers comprising a high concentration of polyphenol antioxidants.

This is achieved by the invention by a potato tuber, comprising a flesh colour ranging from white to dark yellow and an increased concentration of polyphenol antioxidants, in particular flavonoids and chlorogenic acid.

According to the invention, the phenotype comprising a combination of a flesh colour ranging from white to dark yellow and an increased concentration of polyphenol antioxidants is the result of a genotype comprising a gene encoding a tuber anthocyanin pathway activating transcription factor, in particular an R2R3-MYB transcription factor, and lacking a functional flavonoid 3',5'-hydroxylase (F3'5'H) gene and lacking functional dihydrokaempferol-using alleles of a dihydroflavonol 4-reductase (DFR) gene.

In a further embodiment the flesh is orange instead of a colour ranging from white to dark yellow. The potato tuber is then capable of producing high zeaxanthin. Zeaxanthin is a carotenoid found in the cells of the human eye with potent antioxidant properties and links to several health benefits, such as reducing the risk of age-related macular degeneration (AMD), glaucoma, and cataracts. This embodiment is interesting in the light of creating an even more highly biofortified food.

By modulating the biosynthesis of these key antioxidants, it is possible to enhance their concentration in the tubers. The invention thus not only adds nutritional value to the potato tubers but also positions these modified potatoes as a functional food with potential health promoting applications, making them a valuable addition to the diet for promoting overall health.

According to the invention, the term " tuber anthocyanin pathway activating transcription factor" as used herein is intended to refer to any transcription factor that is capable of activating the pathway for anthocyanin production in potato tubers. It is thus a transcription factor that is active in potato tuber flesh. In practice, the term refers to a transcription factor that is located in the potato genome at a locus that is detectable as the HEX signal of the FleshColor-KASP marker shown below:
FAM-GGATCATCACATCACATTCTAAAAT
HEX-GGATCATCACATCACATTCTAAAAC
COM:GTGTGGAGCCTAATAATTAGGCAAACA
In a particular preferred embodiment, the tuber anthocyanin pathway activating transcription factor is an R2R3-MYB transcription factor.

The tuber anthocyanin pathway activating transcription factor, in particular the R2R3-MYB transcription factor, is included to activate the pathway for anthocyanin production so that the redirection thereof leads to an actual accumulation of the desired antioxidant flavonoids. The production of chlorogenic acid is increased as a result of activating the pathway.

The redirection towards flavonoids is achieved by preventing the production of the coloured anthocyanins resulting in colourless flavonoid synthesis. This is done by preventing the use of dihydrokaempferol by either DFR or F3'5'H or both. Many different null-alleles of the F3'5'H gene are present in the cultivated potato germplasm, and many cultivars are therefore unable to produce purple anthocyanins. According to the invention the conversion of DHK into DHM is essentially prevented because the genotype comprises no functional F3'5'H alleles. When there is no DHM as a substrate for DFR no purple coloured anthocyanins are produced and the potato flesh colour remains white or yellow.

The DFR enzyme in cultivated potato is present in two different alleles that display a different substrate-specificity. Most DFR alleles preferentially use DHM as a substrate, but one allele is known to be able to use DHK itself as a substrate, thereby creating pelargonidin, a key precursor to red anthocyanins (Zhang et al. (2009) TAG. Theoretical and Applied Genetics. Theoretische Und Angewandte Genetik, 119(5), 931-937). The potato plant of the invention thus comprises solely alleles encoding dihydroflavonol 4-reductase (DFR) that use dihydromyricetin (DHM) but not dihydrokaempferol (DHK) as a substrate.

The potato plant that lacks dihydrokaempferol-using alleles of the dihydroflavonol 4-reductase (DFR) gene, can either have alleles that use another substrate (DHM) but can also completely lack a functional DFR so that neither DHM nor DHK can be used. If the plant lacks a functional gene this can also mean that it has a defective gene and all alleles are null alleles. Such completely defective DFR gene can also be part of the genotype of the potato tubers and plants of the invention. A null allele is any allele leading to a non-functional version of the encoded enzyme or the complete absence of the encoded enzyme. A null allele could for example comprise a premature stop codon leading to a truncated, non-active version of the enzyme or the null allele may have a mutation in the promotor leading to the gene not being transcribed.

According to the invention a genotype is now created in which both any functional allele of F3'5'H is absent and the DHK-using alleles of DFR are absent or non-functional. As a result, no or only very limited amounts of anthocyanin are produced even though the anthocyanin pathway is activated by the transcription factor. This genetic configuration leads to a significant reduction or even a complete elimination of anthocyanin production. However, minor pathway leakage may occur, resulting in a perceptible red hue in the flesh and tuber skin due to limited pelargonidin accumulation. Consequently, it is possible to develop potato variants exhibiting pale red flesh with elevated flavonoid concentrations and reddish skin.

A genotype of the potato tuber of the invention that does not comprise a functional F3'5'H gene is in fact a genotype in which all alleles of the F3'5'H gene are non-functional. Multiple non-functional alleles are known and for example selected from the group consisting of StPu1, StPu2, StPu3.1, StPu3.2, StPu4, StPu5, StPu6 (as disclosed in Hoopes et al., Molecular Plant 15(3), 520-536 (2022), Supplemental Table 21). StPU7 is a functional allele as well as StPu8, StPu9 and StPu10.

The potato tuber of the invention comprising an increased content of polyphenol antioxidants, in particular chlorogenic acid and flavonoids, can be easily phenotypically selected because a cross section of the tuber is coloured dark grey to black under UV light when observed using a UV camera as a result of the presence of the increased polyphenol content. Normal white- or yellow-fleshed tubers appear white under UV light when observed with a UV-camera. Chlorogenic acid (CGA) and/or polyphenols/flavonoids absorb UV light, whilst "normal' potato flesh reflects it. Red and purple fleshed tubers also absorb UV.

Potato tubers of the invention have an increased concentration of polyphenol antioxidants, which means that they have a concentration of polyphenols, in particular flavonoids and/or chlorogenic acid, that is increased as compared to potato tubers not having the above defined genotype.

In one embodiment, the flavonoids of which the concentration is increased are flavonols and derivatives thereof, in particular selected from kaempferol and its derivates.

The term "chlorogenic acids" refers to a polyphenol family of esters, including hydroxycinnamic acids (caffeic acid, ferulic acid and p-coumaric acid) with quinic acid. Chlorogenic acid is chemically 5-caffeoylquinic acid, an ester of caffeic acid and quinic acid, but the family comprises chlorogenic acid (CGA) isomers, such as 3-caffeoylquinic acid, 4-caffeoylquinic acid, 5-caffeoylquinic acid, 3,5-dicaffeoylquinic acid, 3,4-dicaffeoylquinic acid, and 4,5-dicaffeoylquinic acid, respectively.

The present invention further relates to food products comprising the potato tubers as claimed. Potatoes are very versatile and can be transformed into a wide variety of food products. The potato tubers of the invention can for example be used to prepare mashed potatoes, fries, in particular French fries, fried potatoes, roasted potatoes, steamed potatoes, boiled potatoes, potato chips, potato crisps, potato slices, potato soup, baked potatoes, rösti, latkes, hash browns, gnocchi, potato wedges, potato skins, potato strings, potato croquettes, pommes duchesses, potato flakes, potato salad, etc.

These potato dishes have different preparation methods or different appearances. Preparation methods comprise for example boiling, steaming, roasting, baking, frying and grilling. The potato food products may come as whole potatoes or parts thereof, such as slices of varying thickness, dices, balls, strips, wedges, but also grated, shredded, pureed or mashed. Fries may have different forms or shapes such as shoestring fries, which are very thin and crispy, steak fries, which are thick-cut fries with a soft interior, curly fries, which are spiral-shaped fries, waffle fries, which are fries cut into a lattice shape or crinkle-cut fries, which have a wavy texture.

The invention relates to food products that are prepared from raw potato or from cooked potato. In some embodiments, the food product is pre-cooked and/or deep-frozen.

The food products prepared from the potato tubers of the invention comprise the beneficial increased concentration of polyphenol antioxidants, in particular flavonoids and chlorogenic acid. The potatoes tubers of the invention were cooked and baked and tasted. It was found that the food products tasted normal without off-tastes or off-flavours. The cooking methods also left the increased concentration of polyphenols completely or at least essentially intact.

The present invention further relates to a method for producing a potato tuber comprising an increased concentration of polyphenol antioxidants, in particular flavonoids and chlorogenic acid, comprising growing a potato plant from a potato seed or a seed potato until it produces potato tubers and harvesting the potato tubers at harvest stage, wherein the potato seed or the seed potato shows a genotype comprising a gene encoding a tuber anthocyanin pathway activating transcription factor, in particular an R2R3-MYB transcription factor, lacking a functional flavonoid 3',5'-hydroxylase (F3'5'H) gene and lacking any dihydrokaempferol-using dihydroflavonol 4-reductase (DFR) gene.

Potatoes can be grown from botanical seeds but are usually grown from seed potatoes, which are in fact smaller sized potatoes comprising one or more buds from which new plants are started.

According to a further aspect thereof the invention relates to a method for producing a potato plant that produces potato tubers as claimed, the method comprising:
a) crossing a red- or purple-fleshed parent potato plant having a gene encoding a tuber anthocyanin pathway activating transcription factor, in particular an R2R3-MYB transcription factor, and lacking a functional F3'5'H gene parent potato plant comprising a flesh colour ranging from white to dark yellow and lacking the functional dihydrokaempferol-using allele of a dihydroflavonol 4-reductase (DFR) gene;
b) selecting from the progeny of the cross a potato plant producing potato tubers with white or yellow flesh, of which a cross section colours dark grey or black under UV light when observed using a UV-camera.

Once a white- or yellow-fleshed potato plant is obtained with the genotype of the invention, this plant can be used as a parent to introgress the high polyphenol antioxidant trait of the invention into other white-, yellow- or orange-fleshed potato plants.

In a further embodiment, the genotype of the potato of the invention may comprise only non-functional DFR alleles and a functional F3'5'H. If the DFR is non-functional DHK and DHM can both not be converted and the pathway is still redirected towards the flavonols and chlorogenic acid.

Ultraviolet (UV) light is a form of electromagnetic radiation of wavelengths of 10 to 400 nanometers (nm), which is shorter than that of visible light. It can be divided into three subcategories, UVA (315 to 400 nm), UVB (280 to 315 nm), and UVC (100 to 280 nm).

Flavonoids show significant absorption in the ultraviolet A (UVA) and ultraviolet B (UVB) region, due to their chemical structure with conjugated double bonds. If a potato tuber has a high concentration of flavonoids a cross section of the tuber will appear grey or black under UV light when observed using a UV-camera since the radiation is absorbed by the flavonoids. Chlorogenic acid also has a UV absorption capability. Using UV light to detect the increased polyphenol concentration is an easy way to detect the increased polyphenol phenotype. The colouration under UV can be observed using a UV camera.

In one embodiment, the genotype of a potato plant producing potatoes comprising a flesh colour ranging from white to dark yellow, of which a cross section exhibits grey or black colouration under UV light when observed using a UV-camera comprises a gene encoding a tuber anthocyanin pathway activating transcription factor, in particular an R2R3-MYB transcription factor, lacks a functional flavonoid 3',5'-hydroxylase (F3'5'H) gene and lacks any dihydrokaempferol-using dihydroflavonol 4-reductase (DFR) gene. The transcription factor is included for activating the synthesis of red and purple anthocyanin pigments in tuber flesh. The F3'5'H enzyme is involved in the hydroxylation of flavonoids, contributing to the formation of specific purple anthocyanin pigments. Absence of a functional F3'5'H gene results in the absence of these purple anthocyanins. The DHK-using allele of the DFR enzyme catalyzes the reduction of dihydrokaempferol to leucoanthocyanidins, intermediates in red anthocyanin biosynthesis. The lack of a DHK-using allele of the DFR gene leads to the absence of these red anthocyanin pigments.

Multiple R2R3-MYB transcription factor genes exist that are involved in anthocyanin pathways in flowers, leaves, stems, or tubers. In the genome of potatoes of the invention, the gene encoding an R2R3-MYB transcription factor may be the StMYB200 or StMYB210 gene, the non-functional flavonoid 3',5'-hydroxylase (F3'5'H) gene comprises only null-alleles of the flavonoid 3',5'-hydroxylase (F3'5'H) gene and the dihydrokaempferol-using allele of the dihydroflavonol 4-reductase (DFR) gene that is absent from the genome is the allele encoding the W5281.2 protein (Zhang (2009), which is quite wide-spread in the cultivated potato germplasm.
The protein sequence of the allele is as follows:

MASEVHAVVDAHSPPKTPTVCVTGAAGFIGSWLVMRLLERGYNVHATVRDPENQKKVK
HLLELPKADTNLTLWKADLAVEGSFDEAIQGCQGVFHVATPMDFESKDPENEVIKPTVRG
VLSIIESCAKANTVKRLVFTSSAGALDVQEDQKLFCDETSWSDLDFIYAKKMTGWMYFVS
KILAEKAAMEEAKKNNIDLISIIPPLVVGPFITPTFPPSLITALSLITGNEAHYGIIKQGQYVHL
DDLCEAHIFLYEHPKAEGRFICSSHHAIIYDVAKMVRQKWPEYYVPTE*

In diploid potatoes, the number of null alleles should be two. In tetraploid potatoes, the number of null-alleles is four. This also means that of the alleles lacking from the genome or non-functional alleles, all alleles should be lacking or be non-functional.

The parent of the cross can be diverse as long as their off-spring has the combination of a gene encoding a tuber anthocyanin pathway activating transcription factor, in particular an R2R3-MYB transcription factor and lacks functional F3'5'H alleles and has no dihydrokaempferol-using dihydroflavonol 4-reductase (DFR) allele or no functional DFR allele. The invention was made by crossing the red-fleshed the cultivar Mulberry Beauty as the one parent potato plant and the white-fleshed cultivar Alverstone Russet as the other parent potato. Other examples of suitable combinations of parents are Vitelotte noir x Colomba, Blue Star x Innovator, Magenta Love x Desireé, All Blue x Spunta, Red Thumb x Russian Blue.

The invention further relates to a method for selecting a potato tuber as claimed, comprising observation of a cross section of a tuber comprising a flesh colour ranging from white to dark yellow under UV light when observed using a UV-camera and selecting the tuber that exhibits a grey or black flesh colour as a potato tuber having an increased concentration of polyphenol antioxidants, in particular flavonoids and chlorogenic acid.

In a further embodiment, a method for selecting a potato tuber as claimed, comprises:
a) crossing a parent potato plant comprising a gene encoding a tuber anthocyanin pathway activating transcription factor, in particular an R2R3-MYB transcription factor, and red or purple flesh with another parent potato plant comprising a flesh colour ranging from white to dark yellow;
b) selecting in the progeny of the cross plants that produce potato tubers comprising a flesh colour ranging from white to dark yellow and carrying the gene encoding the transcription factor; and
c) observing a cross section of a tuber with white or yellow flesh under UV light using a UV-camera and selecting the tuber that exhibits a grey or black colour as a potato tuber having an increased concentration of polyphenol antioxidants, in particular flavonoids and chlorogenic acid.

Alternatively, instead of crossing a parent potato plant comprising a gene encoding a tuber anthocyanin pathway activating transcription factor, in particular an R2R3-MYB transcription factor, and red or purple flesh with another parent potato plant comprising a flesh colour ranging from white to dark yellow, the parent with the transcription factor can have white or yellow flesh and the other parent is red- or purple-fleshed. In further embodiments, the genotype can be detected by selfing purple- or red-fleshed parents or by crossing a red-fleshed parent with another red-fleshed parent, a purple-fleshed parent with another purple fleshed parent or a red-fleshed parent with a purple fleshed parent.

In the present application the flesh colour of the potato tubers of the invention is described as "ranging from white to dark yellow". This means that the potato can have any colour in this spectrum. Examples are white, cream, light yellow, pale yellow, golden, yellow, dark yellow. The potato tubers of the invention do not have a red or purple colour or only a slightly red colour when some leakage occurs.

In one embodiment, the flesh colour of a potato tuber of the invention can even be orange. In that case, however, the flesh comprises high levels of one or more carotenoids, in particular of the xanthophylls lutein and zeaxanthin.

The present invention will be illustrated in the example that follows and that is not intended to limit the invention in any way. Other combinations of parent potato plants can be used provided their progeny comprises the required genotype.

The invention provides potato tubers with a flesh colour ranging from white to dark yellow that still have an increased concentration of health-beneficial compounds that are usually only found in potato tubers with red or purple flesh. The invention shows that the anthocyanin pathway can successfully be redirected towards the synthesis of compounds that are not coloured but still offer potential health benefits. This can also advantageously be done without the need for genetic engineering of the potato plant.

According to a further aspect, the potato tubers of the invention have an increased resistance to particular insects that feed on the tuber, such as wireworms. As a result of the increased polyphenol content of the tubers, they are more resistant to such insects. Polyphenols can deter insects from feeding on potato tubers by making the tuber tissue less palatable or even toxic to the insects. Polyphenols may also contribute to the strengthening of plant cell walls, making it more difficult for insects to penetrate and feed on the plant.

The invention will now be elucidated in the example that follows and that is given for illustration purposes only and are not intended to limit the invention in any way.

In the Example, reference is made to the following figures:
Fig. 1 shows a schematic representation of the anthocyanin pathway
**Fig. 2** shows the white-fleshed parent Alverstone Russet (left), and the red-fleshed parent Mulberry Beauty (right), using a normal camera (a) and using a UV-camera (b)
**Fig. 3** shows the phenotypes identified in the segregating population from the crossing between Alverstone Russet x Mulberry Beauty
**Fig. 4** shows two halves of two white fleshed potatoes (a), where the left tuber is reflecting all UV light and colours white when observed using a UV camera, while the right one absorbs UV light (b) and exhibits a dark grey colour when observed using a UV-camera
**Fig. 5** shows the chlorogenic acid content in potato flesh that is purple (a), red (b), white/yellow (c), and UV-absorbing (d)
**Fig. 6** shows the content of kaempferol derivates in potato flesh that is purple (a), red (b), white/yellow (c), and UV-absorbing (d)
**Fig. 7** shows that UV-absorption is independent of white or yellow flesh

### EXAMPLE

### Materials and methods

A cross was made between the red-fleshed cultivar Mulberry Beauty and the white-fleshed cultivar Alverstone Russet (both obtainable from HZPC) **(****Fig. 1****).** The genotype of Mulberry Beauty is as follows: DFR-DHK, 1, functional P-locus, 0, R2R3-MYB tuber TF, 1. The genotype of Alverstone Russet is: DFR-DHK, 0, functional P-locus, 1, R2R3 MYB tuber TF, 0. The P-locus encodes the flavonoid 3',5'-hydroxylase (F3'5'H) gene. In Alverstone Russet one of the four alleles was a functional allele, StPu7.

Pre-screening of tuber flesh was done using an UVLOOKmini UV-camera for smartphone to select potential progeny plants of the invention. Progeny plants of the invention show dark-grey or black flesh under UV.

HPLC - LC/MS was used for the determination and quantification of compounds present in the potato flesh. These include derivates of kaempferol, such as kaempferol 3-O-sophoroside, kaempferol 3,7-di-O-glucoside, kaempferol 3-(2G-glucorutinoside), and also isomers of chlorogenic acids, such as 3-O-caffeoylquinic acid, 4-O-caffeoylquinic acid, and 5-O-caffeoylquinic acid. These are also detected in purple and red fleshed potatoes, albeit at lower concentrations. In normal white and yellow fleshed potatoes these compounds are almost completely absent.

Potatoes were steamed or fried, and tasted.

KASP-markers for the DHK-using allele of DFR, StPu7, and for the tuber anthocyanin-pathway-activating transcription factor (sequences shown below) were run using PACE-mix, 40 cycles, under standard KASP-conditions. The HEX signal signifies the presence of StPu7, the tuber anthocyanin pathway activating transcription factor and the DHK-using allele of DFR, respectively.
StPu7-KASP (marker for the functional F3'5'H allele StPu7)
   HEX-TCTTCTTTTTTGTGTTATTGAGTAAATTTTT
   FAM-TCTTCTTTTTTGTGTTATTGAGTAAATTTC
   COM:AGTTGGAAGTAACGCATTAATACAGGATTC
FleshColor-KASP (marker for tuber anthocyanin pathway activating transcription factor)
   FAM-GGATCATCACATCACATTCTAAAAT
   HEX-GGATCATCACATCACATTCTAAAAC
   COM:GTGTGGAGCCTAATAATTAGGCAAACA
DFR-DHK-KASP (marker for the DHK-using allele of DFR)
   FAM-GGTTTTCACTTCATCTGCTGGAA
   HEX-GGTTTTCACTTCATCTGCTGGAG
   COM:GAGTTTTTGGTCCTCTTGKACATCAA

### Results

Genotypes that scored negative on HEX signal for all three KASP-markers were white or yellow fleshed, and did not absorb UV. Genotypes that scored positive for StPu7 and/or DFR-DHK, but negative for FleshColor were also white or yellow fleshed, without UV absorption. Genotypes that scored positive for FleshColor and DFR-DHK, but negative for StPu7, were red-fleshed, and absorb UV. Genotypes that score positive for FleshColor and StPu7 (regardless of DFR-DHK score) were purple fleshed, and absorb UV. Only genotypes that scored negative for both StPu7 and DFR-DHK, but positive for FleshColor, showed white or yellow flesh while absorbing UV light.

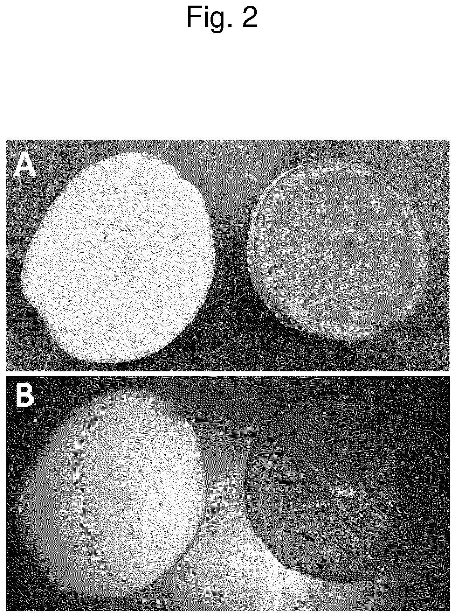

**Fig. 2** shows that the flesh of parent Mulberry Beauty absorbs UV-light, while the flesh of parent Alverstone Russet does not. This shows the presence of UV-absorbing compounds in the flesh of Mulberry Beauty, but not in the flesh of Alverstone Russet.

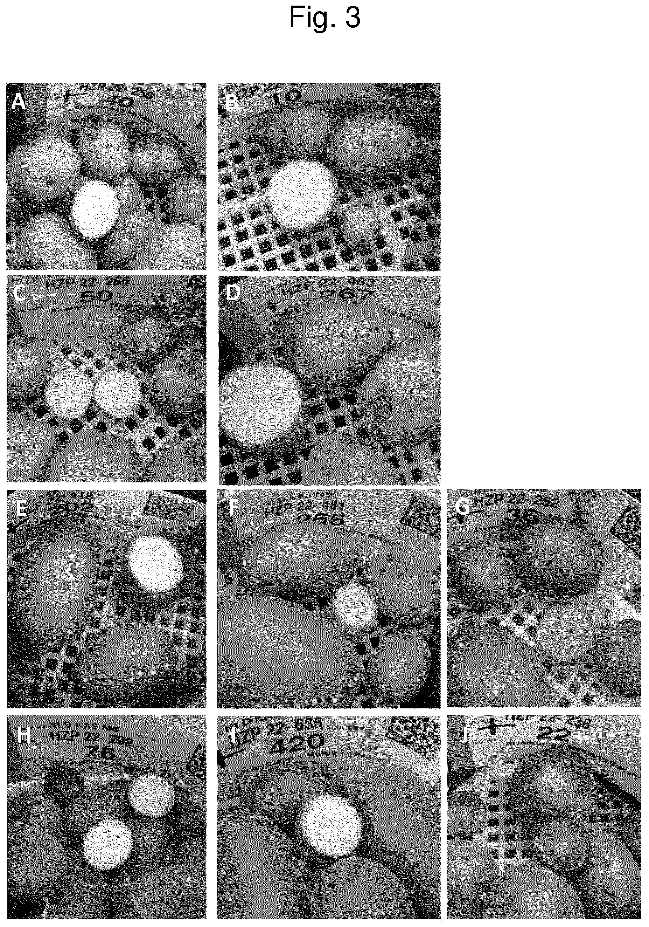

Initially, the cross between Alverstone Russet x Mulberry Beauty led to a segregating population with phenotypes ranging from yellow skin, white flesh (a), yellow skin, yellow flesh (b), slightly reddish skin, white flesh (c), slightly red skin, yellow flesh (d), red skin, white flesh (e), red skin, yellow flesh (f), red skin, red flesh (g), purple skin, white flesh (h), purple skin, yellow flesh (i), and purple skin, purple flesh (j). This is shown in Figure 3.

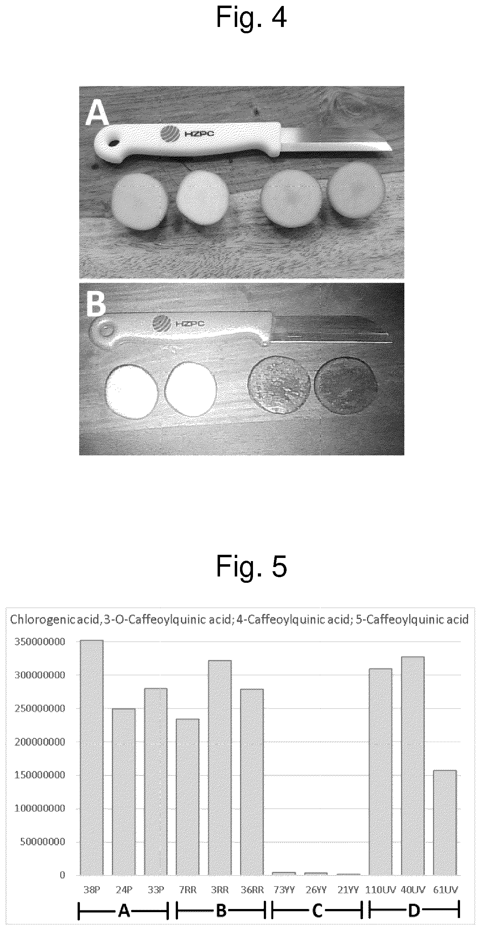

**Fig. 4** shows the tubers of a normal potato with white flesh and no increased polyphenol concentration (left) in comparison to a potato of the invention (right) which has also white flesh but does absorb UV as a result of an increased polyphenol concentration.

**Fig. 5** shows that the levels of chlorogenic acids in the flesh of the UV-absorbing potatoes are comparable to purple and red fleshed potatoes, and overall, much higher than in normal white/yellow fleshed potatoes. Potatoes 38P, 24P and 33P have purple flesh. 7RR, 3RR and 36RR are red. 73YY, 26YY and 21 YY are yellow. 110UV, 40UV and 61 UV are potato tubers of the invention.

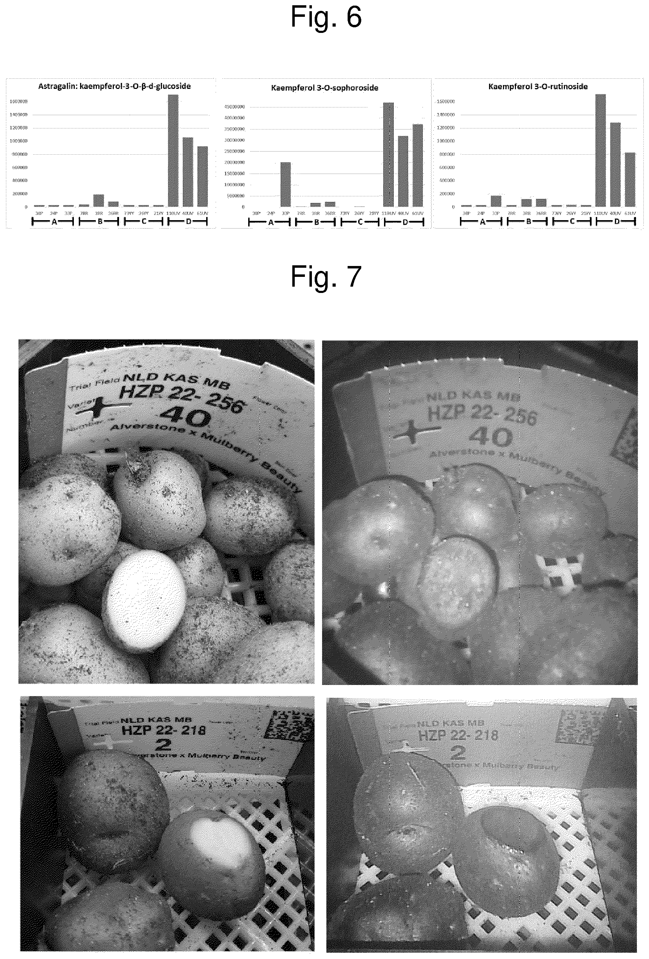

**Fig. 6** shows the content of various flavonoids in the same potatoes. It can be seen that the content is higher in potato tubers of the invention.

In **Fig. 7** it is shown that the UV absorption is the same in potato tubers with white flesh (top) as in tubers with yellow flesh (bottom).

In the steamed and fried potato tubers no off-smell or off-taste could be detected.

## Claims

1. Potato tuber, comprising a flesh colour ranging from white to dark yellow and an increased concentration of polyphenol antioxidants, in particular flavonoids and chlorogenic acid.

2. Potato tuber as claimed in claim 1, wherein the combination of a flesh colour ranging from white to dark yellow and an increased concentration of polyphenol antioxidants is the result of a genotype comprising a gene encoding a tuber anthocyanin pathway activating transcription factor, in particular an R2R3-MYB transcription factor, and lacking a functional flavonoid 3',5'-hydroxylase (F3'5'H) gene and lacking functional dihydrokaempferol-using alleles of a dihydroflavonol 4-reductase (DFR) gene.

3. Potato tuber as claimed in claim 2, wherein all alleles of the F3'5'H gene are non-functional and selected from the group consisting of StPu1, StPu2, StPu3.1, StPu3.2, StPu4, StPu5 and StPu6.

4. Potato tuber as claimed in any one of the claims 1 to 3, wherein a cross section of the tuber is coloured dark grey to black under UV light when observed with a UV camera.

5. Potato tuber as claimed in any one of the claims 1 to 4, wherein the concentration of polyphenols, in particular colourless flavonoids and/or chlorogenic acid, is increased as compared to potato tubers not having the genotype as defined in claim 2.

6. Potato tuber as claimed in any one of the claims 1 to 5, wherein the flavonoids of which the concentration is increased are flavonols, in particular kaempferol and its derivatives, such as kaempferol 3-O-sophoroside, kaempferol 3,7-di-O-glucoside, kaempferol 3-(2G-glucorutinoside).

7. Food product, comprising a potato tuber as claimed in any one of the claims 1 to 6 or a part thereof, optionally in processed form.

8. Food product as claimed in claim 7, which is selected from the group consisting of mashed potatoes, fries, in particular French fries, fried potatoes, roast potatoes, steamed potatoes, boiled potatoes, potato chips, potato crisps, potato slices, potato soup, baked potato, rösti, latkes, hash browns, gnocchi, potato wedges, potato strings, potato skins, potato flakes, potato croquettes, pommes duchesses, wherein the food product is optionally pre-cooked and/or deep-frozen.

9. Method for producing a potato tuber as claimed in any one of the claims 1 to 6, comprising growing a potato plant from a potato seed or a seed potato until it produces potato tubers and harvesting the potato tubers at harvest stage, wherein the potato seed or the seed potato shows a genotype comprising a gene encoding a tuber anthocyanin pathway activating transcription factor, in particular an R2R3-MYB transcription factor, lacking a functional flavonoid 3',5'-hydroxylase (F3'5'H) gene and lacking functional dihydrokaempferol-using alleles of a dihydroflavonol 4-reductase (DFR) gene.

10. Method for producing a potato plant that produces potato tubers as claimed in any one of the claims 1 to 6, comprising:
a) crossing a red-fleshed parent potato plant having a gene encoding a tuber anthocyanin pathway activating transcription factor, in particular an R2R3-MYB transcription factor, and lacking a functional F3'5'H gene with a parent potato plant comprising tubers with a flesh colour ranging from white to dark yellow and lacking functional dihydrokaempferol-using alleles of a dihydroflavonol 4-reductase (DFR) gene;
b) selecting from the progeny of the cross a potato plant producing potato tubers with a flesh colour ranging from white to dark yellow, of which a cross section colours dark grey or black under UV light, when observed using a UV-camera.

11. Method as claimed in claim 10, wherein the potato plant producing potato tubers with a flesh colour ranging from white to dark yellow, a cross section of which colouring dark grey or black under UV light, when observed using a UV-camera has a genotype comprising a gene encoding a tuber anthocyanin pathway activating transcription factor, in particular an R2R3-MYB transcription factor, lacking a functional flavonoid 3',5'-hydroxylase (F3'5'H) gene and lacking functional dihydrokaempferol-using alleles of a dihydroflavonol 4-reductase (DFR) gene.

12. Method as claimed in claim 11, wherein the gene encoding a tuber anthocyanin pathway activating transcription factor, in particular an R2R3-MYB transcription factor, is the StMYB200 or StMYB210 gene, the non-functional flavonoid 3',5'-hydroxylase (F3'5'H) gene comprises solely null-alleles of the flavonoid 3',5'-hydroxylase (F3'5'H) gene and the dihydrokaempferol-using allele of a dihydroflavonol 4-reductase (DFR) gene that is absent from the genome is the red allele encoding the W5281.2 protein.

13. Method as claimed in any one of the claims 10-12, wherein the red-fleshed parent potato plant is the cultivar Mulberry Beauty and the white-fleshed parent is the cultivar Alverstone Russet.

14. Method for selecting a potato tuber as claimed in any one of the claims 1 to 6, comprising observation of a cross section of a tuber with white or yellow flesh under UV light using a UV-camera and selecting the tuber that colours dark grey or black as a potato tuber having an increased concentration of polyphenol antioxidants, in particular flavonoids and chlorogenic acid.

15. Method for selecting a potato tuber as claimed in any one of the claims 1 to 6, comprising:
a) crossing a parent potato plant comprising a gene encoding a tuber anthocyanin pathway activating transcription factor, in particular an R2R3-MYB transcription factor, and red or purple flesh with another parent potato plant comprising flesh ranging from white to dark yellow;
b) selecting in the progeny of the cross plants that produce potato tubers with flesh ranging from white to dark yellow and carrying the gene encoding the transcription factor; and
c) observing a cross section of a tuber with flesh ranging from white to dark yellow using a UV-camera and selecting the tuber that colours dark grey or black as a potato tuber having an increased concentration of polyphenol antioxidants, in particular flavonoids and chlorogenic acid.
